# EUROPÄISCHE PATENTSCHRIFT

(11) **EP 2 317 773 B1**
(45) Veröffentlichungstag und Bekanntmachung des Hinweises auf die Patenterteilung: **11.07.2012**
(21) Anmeldenummer: 10186511.1
(22) Anmeldetag: 05.10.2010
(51) Int. Cl.: H04R 1/10, A61F 11/08, H04R 29/00, H04R 25/00

(54) **Verfahren zum Überprüfen einer Passform einer ein schallregulierendes Element aufweisenden Otoplastik in einem menschlichen Ohr**
Method for inspecting the fit of an otoplastic comprising an acoustic regulation element in a human ear
Procédé de contrôle d'un ajustement d'une prothèse auditive dotée d'un élément régulant le son dans une oreille humaine

(30) Priorität: 16.10.2009 AT 16322009
(43) Veröffentlichungstag der Anmeldung: 04.05.2011
(73) Patentinhaber: Audio Lab Austria GmbH, 8421 Schwarzau im Schwarzautal (AT)
(72) Erfinder: Thanner, Christoph, 4600 Wels (AT)
(74) Vertreter: Wirnsberger, Gernot

(56) Entgegenhaltungen:
- EP-A1- 1 071 307
- WO-A1-2005/055656
- US-A- 4 974 606
- US-A1- 2006 045 297
- US-A1- 2008 011 084

## Beschreibung

Die Erfindung betrifft ein Verfahren zum Überprüfen einer Passform einer mit einem lösbar befestigten, einen Schalldurchtritt regulierenden Element ausgestatteten Otoplastik in einem menschlichen Ohr bzw. Gehörgang.

Arbeiter in Industriebetrieben sind, neben anderen Umwelteinflüssen, grundsätzlich auch einem großen Lärm ausgesetzt, welcher durch Produktionsanlagen verursacht wird. Um die Arbeiter vor dauerhaften gesundheitlichen Schäden zu schützen, werden verschiedene Lärmschutzsysteme angeboten, die an bzw. in den Ohrmuscheln oder in den Gehörgängen getragen werden.

Unter den bekannten Lärmschutzsystemen sind insbesondere sogenannte Otoplastiken bevorzugt. Otoplastiken weisen menschlichen Ohren bzw. Gehörgängen angepasste äußere Formen bzw. Konturen auf und können in den Ohren bzw. Gehörgängen trotz kleiner Bauweise effektiv einen Schalldurchtritt reduzieren und somit Lärmbeeinträchtigungen verhindern. Diese Otoplastiken sind häufig mit Ventilen ausgestattet, die es erlauben, eine Intensität des durchtretenden Schalls zu regulieren. Alternativ zu Ventilen kommen andere Elemente zur Schallregulierung zum Einsatz, z. B. Steckfilter, die in beispielsweise zentral verlaufende Öffnungen von Otoplastiken eingesetzt werden. Hintergrund für diese konstruktive Maßnahme ist, dass die Otoplastiken einerseits menschliche Ohren vor unerwünscht hoher Lärmbeeinträchtigung schützen sollen, andererseits aber auch sichergestellt sein soll, dass eine gewisse Schalldurchlässigkeit gegeben ist, die beispielsweise eine Kommunikation mit Arbeitskollegen oder insbesondere ein akustisches Erkennen von Warnsignalen, beispielsweise bei Ausfällen von Maschinen im Produktionsbetrieb, erlaubt. Durch die maßgenaue Passform ist ein mehrstündiges Tragen der Otoplastiken möglich.

In der Praxis werden für jeden Benutzer individuell angepasste Otoplastiken z. B. mit voreingestellten Ventilen ausgeliefert. Die individuelle Anpassung erfolgt durch Anfertigen von Abdrücken der Ohren bzw. Gehörgänge des Benutzers und anschließend entsprechende konturgetreue Ausformung der Otoplastiken im Außenbereich. Die Ventile sind ab Werk so eingestellt, dass eine Kommunikation über kurze Distanzen oder ein Erkennen von lauten Warnsignalen möglich ist, Maschinengeräusche jedoch für das menschliche Ohr grundsätzlich unterdrückt werden bzw. meist durch Normen vorgegebene Dämmungswerte erreicht werden. Anschließend werden die Otoplastiken versiegelt und in diesem Zustand dem Benutzer übergeben. Nach Auslieferung werden die Otoplastiken bei der Arbeit im Betrieb teilweise mehrere Jahre verwendet. Kommen andere schallregulierende Elemente, beispielsweise Steckfilter, zum Einsatz, verhält es sich ähnlich.

Die die Ventile oder allgemein schallregulierenden Elemente umgebenden äußeren und mit einem Ohr bzw. Gehörgang in Kontakt stehenden Teile der Otoplastiken sind in der Regel aus einem Kunststoff gebildet. Obwohl die eingesetzten Kunststoffe durchaus hohe Qualitäten haben und nach langer Tragedauer der Otoplastiken eine zumindest nahezu unveränderte Kontur aufweisen und auch die Ventile sich im Idealfall wie im Werk eingestellt verhalten, kann es doch vorkommen, dass sich die Konturen der menschlichen Ohren bzw. Gehörgänge geringfügig ändern und die Otoplastiken deshalb nicht mehr perfekt sitzen. Daher ist eine Passform der Otoplastiken regelmäßig zu überprüfen, um sicherzustellen, dass auch nach längerer Tragedauer mit den Otoplastiken vorgegebene Dämmungswerte erreicht werden.

In der WO 2005/055656 A1 ist ein Verfahren zur Bestimmung der Dichtigkeit einer Otoplastik offenbart, wobei ein Adapter mit zwei schalltechnisch getrennten Mikrofonen fest in einen Hörkanal einer Otoplastik eingesetzt wird, wobei ein Mikrofon außenseitig ist und ein Mikrofon innenseitig im Hörkanal Schall aufnimmt. Vereinfacht dargestellt wird aus einer Differenz von Messungen mit den Mikrofonen außenseitig und innenseitig eine Dichtigkeit der Otoplastik bestimmt. Das Verfahren erfordert jedoch nicht nur mehrere Mikrofone, sondern auch ein genaue Kalibrierung.

In der EP 1 071 307 A1 ist eine Otoplastik offenbart, die einen mit einem Filter ausgestatteten Hörkanal aufweist, der nach außen hin auch mit einer Aufnahmeöffnung in Verbindung steht. In die Aufnahmeöffnung kann ein Lautsprecher oder ein schalldicht abschließendes Element eingesetzt werden.

Die bekannten Verfahren zur Überprüfung der Passform einer Otoplastik weisen den Nachteil auf, dass die Verfahren äußerst aufwendig sind. Darüber hinaus haftet vielen

Verfahren der Nachteil an, dass diese nicht zu genauen und zuverlässigen Ergebnissen führen.

Aufgabe der Erfindung ist es, diese Nachteile des Standes der Technik zu beseitigen.

Die der Erfindung zugrunde liegende Aufgabe wird dadurch gelöst, dass ein Verfahren der eingangs genannten Art folgende Schritte umfasst:
a) Entnehmen des Elementes aus der Otoplastik;
b) Befestigen eines Messadapters mit einem dem Ohr bzw. Gehörgang zugewandten Mikrofon in einer Position des Elementes derart, dass der Messadapter lose sitzt und Schall zwischen dem Messadapter (8) und der Otoplastik (1) ungehindert durchtreten kann;
c) Beschallen der Otoplastik und Aufnehmen eines Referenzspektrums mit zumindest einem Prüfsignal;
d) Befestigen des Messadapters in der Otoplastik derart, dass der Messadapter fest anliegt und kein Schall zwischen dem Messadapter (8) und der Otoplastik (1) durchdringen kann;
e) Beschallen der Otoplastik und Aufnehmen eines Messspektrums unter den gleichen Bedingungen wie beim Aufnehmen des Referenzspektrums;
f) Bilden einer Differenz aus dem Messspektrum abzüglich des Referenzspektrums und Ermitteln der Passform der Otoplastik anhand der Differenz;
wobei das Messspektrum auch vor dem Referenzspektrum aufgenommen werden kann.

Die mit der Erfindung erzielten Vorteile sind insbesondere darin zu sehen, dass auf besonders einfache Weise rasch und zuverlässig die aufgrund der Passform gegebene Dichtheit der Otoplastik im Ohr bzw. Gehörgang eines Benutzers festgestellt werden kann. Dazu wird das schallregulierende Element aus der Otoplastik entfernt und ein den Außenabmessungen des Elementes entsprechender Messadapter mit einem Mikrofon in die Otoplastik eingesetzt, wobei das Mikrofon, in Richtung des Gehörganges betrachtet, dem Gehörgang zugewandt ist. Dabei erfolgt ein Positionieren des Messadapters vorerst so, dass dieser in der Otoplastik lose befestigt ist. Anschließend wird die Otoplastik und damit der Messadapter beschallt, indem beispielsweise über einen Kopfhörer ein entsprechender Schall aufgebracht wird. Zwar sitzt der Messadapter in der Otoplastik, da aber dessen Sitz lose ist, tritt Schall zwischen dem Messadapter und der Otoplastik ungehindert durch, sodass in dieser Weise ein Referenzspektrum aufgenommen werden kann, das die Messbedingungen nahezu perfekt repräsentiert. Anschließend wird der Messadapter mit dem Mikrofon fest anliegend in der Otoplastik befestigt. In dieser Position des Messadapters kann kein Schall zwischen diesem und der Otoplastik durchdringen. Das bei Aufbringen eines Schalls nunmehr gemessene Spektrum, das Messspektrum, zeigt daher lediglich jenen Schall, der aufgrund eines nicht perfekten Sitzes der Otoplastik als solcher im Ohr bzw. Gehörgang gemessen wird. Dieses Messspektrum ist durch Bilden einer Differenz um das Referenzspektrum zu korrigieren, damit der zwischen Otoplastik und Ohr bzw. Gehörgang durchtretende Schall unverfälscht ermittelt werden kann. Da für die beiden Messungen, inklusive eines Hantierens an dem Messadapter, lediglich 60 Sekunden oder weniger benötigt werden, kann in einfacher und rascher Weise eine Passform der Otoplastik festgestellt werden. Sofern die Otoplastik nicht in der geforderten Weise dichtend sitzt, ist diese durch eine neue Otoplastik zu ersetzen. Darüber hinaus können mit dem erfindungsgemäßen Verfahren auch Leckstellen in der Otoplastik selbst nachgewiesen werden, z. B. ein Riss im das schallregulierende Element umgebenden Kunststoff.

Es versteht sich im Rahmen der Erfindung, dass das Messspektrum auch vor dem Referenzspektrum aufgenommen werden kann.

Sofern die Otoplastik nicht optimal sitzt, zeigt sich dies insbesondere bei niedrigen Frequenzen. Es ist daher ausreichend, wenn die Otoplastik mit einem Prüfsignal mit einer Frequenz von weniger als 300 Hz beschallt wird und die mit diesem Prüfsignal ermittelte Differenz zur Qualitätsbeurteilung herangezogen wird. Eine besonders geeignete Frequenz liegt um 250 Hz.

Das Beschallen kann, wie bereits erwähnt, mittels eines Kopfhörers durchgeführt werden.

Wenngleich das Beschallen bevorzugt mit einer einzigen Frequenz erfolgt, kann doch auch vorgesehen sein, dass mehrere Prüfsignale verschiedener Frequenzen angewandt werden und aus der Differenz ein Mittelwert gebildet wird, der mit einem vorgegebenen Mittelwert bzw. Qualitätskriterium für die Passform der Otoplastik verglichen wird. Sofern der Mittelwert dem Qualitätskriterium nicht entspricht, ist die Otoplastik auszutauschen. Alternativ ist es möglich, bei jeder einzelnen Frequenz eine Differenz zu ermitteln, die mit einem vorgegebenen Sollwert bzw. Sollbereich verglichen wird.

Weitere Merkmale, Vorteile und Wirkungen der Erfindung ergeben sich aus dem nachfolgend dargestellten Ausführungsbeispiel, anhand dessen die Erfindung noch weitergehend erläutert ist. Soweit dabei auf die anliegenden Zeichnungen Bezug genommen wird, zeigen in diesen:
Fig. 1 eine Otoplastik in teilweise querschnittlicher Darstellung;
Fig. 2 einen inneren Teil bzw. eine Hülse einer Otoplastik;
Fig. 3 einen Schnitt entlang der Linie III-III in Fig. 2;
Fig. 4 ein in eine Hülse einsetzbares Ventil im Querschnitt;
Fig. 5 eine schematische Darstellung eines Messvorgangs;
Fig. 6 eine Darstellung von Spektren bzw. daraus erhaltenen Messergebnissen.

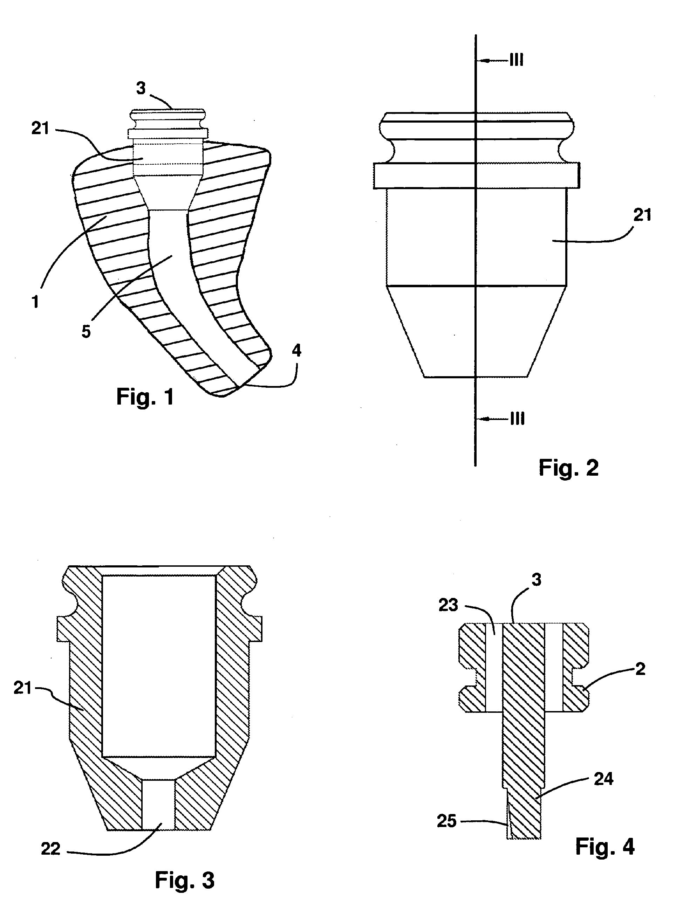

Fig. 1 bis Fig. 4 zeigen eine Otoplastik 1 und einen inneren Teil bzw. eine Hülse 21 derselben sowie ein in diese einsetzbares schallregulierendes Element 2 in Form eines Ventils. Anstelle des Ventils könnte z. B. auch ein Steckfilter oder ein anderes schallregulierendes Element vorgesehen sein. Die Otoplastik 1 ist außenseitig in der Regel aus einem Kunststoff geformt, welcher die Hülse 21 umgibt, wobei eine äußere

Oberfläche bzw. Kontur der Otoplastik 1 exakt einem menschlichen Ohr bzw. Gehörgang angepasst ist, was nicht näher dargestellt ist. Die Hülse 21 der Otoplastik 1 ist im Zentrum im Wesentlichen hohl ausgebildet und weist einen inneren Kanal 22 auf, der einen Sitz für ein Ventil bildet, welches in die Otoplastik 1 eingeschraubt werden kann. Sofern sich der die Hülse 21 umgebende Kunststoff zum Gehörgang hin über den inneren Kanal 22 erstreckt, was in Fig. 1 dargestellt, aber nicht zwingend ist, schließt an den Kanal 22 der Hülse 21 im Kunststoff ein Kanal 5 an.

Das schallregulierende Element 2 bzw. Ventil ist an einem Ende mit einem Zapfen 24 ausgebildet, der eine Nut 25 aufweist. Eine Schalldurchlässigkeit der Otoplastik 1 von einer Außenseite 3 zu einer Innenseite 4, die beim Tragen dem menschlichen Gehörgang zugewandt ist, kann durch Justieren des Ventils stufenlos eingestellt werden. Je weiter das Ventil in den Kanal 22 eingebracht ist, desto mehr schließt sich ein freier Querschnitt der zur Außenseite 3 hin verjüngt ausgebildeten Nut 25 und umso geringer ist eine Schalldurchlässigkeit der Otoplastik 1 von der Außenseite 3 zur Innenseite 4 entlang von Durchlässen 23, der Nut 25 und des Kanals 5. Eine Hülse 21 für das Ventil ist von Vorteil, denkbar ist es aber auch, das Ventil wie auch andere schallregulierende Elemente, z. B. Steckfilter, unmittelbar in der Otoplastik 1 anzuordnen.

Vor Auslieferung einer Otoplastik 1 wird in diese bzw. deren Hülse 21 ein Ventil lösbar eingesetzt, z. B. durch Einschrauben, und auf eine vorgegebene Position eingestellt, sodass eine gewisse, aber nicht allzu hohe Schalldurchlässigkeit der Otoplastik 1 gegeben ist. Die Schalldurchlässigkeit soll so gewählt sein, dass z. B. laute Warnsignale hörbar sind, störende Maschinengeräusche aber nicht durch die Otoplastik 1 dringen können. Quantitativ kann dies durch Normen festgelegt sein, die für bestimmte Frequenzen bestimmte Dämmungswerte und/oder -bereiche vorschreiben.

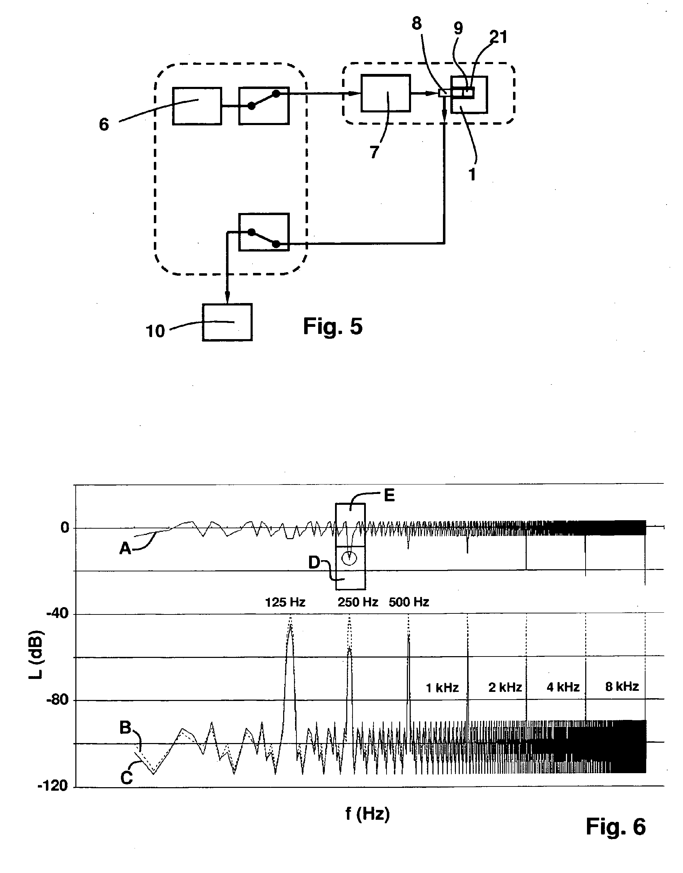

Außenseitig ist die Otoplastik 1 exakt einem menschlichen Ohr bzw. Gehörgang angepasst. Dies wird erreicht, indem vor einem Erstellen der Otoplastik 1 ein Abdruck des menschlichen Ohres bzw. Gehörganges angefertigt und die Otoplastik 1 entsprechend der Kontur des Abdruckes hergestellt wird. Nach langer Tragezeit kann es allerdings dazu kommen, dass sich das menschliche Ohr bzw. der menschliche Gehörgang auch aufgrund eines ständigen Tragens der Otoplastik 1 verändert, sodass die Otoplastik 1 nicht mehr optimal bzw. im Kontaktbereich dichtend im Ohr bzw. Gehörgang sitzt. Zur Überprüfung, ob dies der Fall ist, wird eine Messung entsprechend einem Schema gemäß Fig. 5 durchgeführt. Dabei wird vorerst das Ventil aus der Otoplastik 1 entfernt. Anstelle des Ventils wird ein Messadapter 8 mit einem Mikrofon 9 in die Hülse 21 eingeführt, und zwar so, dass der Messadapter 8 lose sitzt. Anschließend wird die Otoplastik 1 bzw. der Messadapter 8 beschallt. Dies kann erfolgen, indem durch einen Signalgenerator 6 ein Signal erzeugt und dieses durch einen Schallwandler umgewandelt wird. Beispielsweise kann ein Kopfhörer 7 zum Beschallen der Otoplastik 1 eingesetzt werden. Die mit dem Mikrofon 9 aufgenommenen Schallintensitäten werden über eine Auswerteeinheit 10 ausgewertet und digital gespeichert. Da der Messadapter 8 bei dieser Referenzmessung bereits in der Otoplastik 1 bzw. der Hülse 21 sitzt, entspricht das so aufgenommene Referenzspektrum im Wesentlichen genau jenen Messbedingungen, die bei der Aufnahme eines Messspektrums zu berücksichtigen sind, um ein nahezu unverfälschtes Messergebnis zu erhalten. Dieses Messspektrum wird im Anschluss aufgenommen, indem vorerst der Messadapter 8 fest anliegend in der Otoplastik 1 befestigt wird und anschließend ein Beschallen unter, in Bezug auf das Referenzspektrum, identen Bedingungen vorgenommen wird. Dabei kann nur mehr zwischen der Otoplastik 1 und dem Ohr bzw. Gehörgang durchtretender Schall das Mikrofon 9 erreichen. Anschließend wird eine Differenz aus dem Messspektrum abzüglich des Referenzspektrums gebildet. Selbstverständlich ist es auch möglich, zuerst das Messspektrum aufzunehmen und anschließend den Messadapter 8 zu lockern und das Referenzspektrum aufzunehmen.

Fig. 6 zeigt ein Messergebnis B, das einem Referenzspektrum entspricht, ein Messergebnis C, das einem Messspektrum entspricht, sowie ein Differenzspektrum A, das der Differenz des Messspektrums abzüglich des Referenzspektrums entspricht. Aus dieser Differenz kann für verschiedene Frequenzbereiche ermittelt werden, ob und mit welcher Intensität Schall zwischen der Otoplastik 1 und dem Ohr bzw. Gehörgang durchtreten kann. Für viele Zwecke ist es ausreichend, dass lediglich im Bereich einer Frequenz von etwa 250 Hz eine Durchlässigkeit von Schall ermittelt wird. Zur raschen Überprüfung kann dabei ein Schwellwert vorgegeben werden, anhand dessen die Passform bzw. Dichtheit der Otoplastik 1 als geeignet bzw. als ungeeignet bewertet wird, wie es in Fig. 6 mit den Bereichen D, E angedeutet ist, wobei Bereich D einem geeigneten und Bereich E einem ungeeigneten Bereich des Dämmungswertes der Otoplastik 1 entspricht. Damit lässt sich das in Fig. 6 durch einen Kreis hervorgehobene Messergebnis auf einem Bildschirm eines Computers rasch als geeignet bzw. ungeeignet darstellen, wobei die Bereiche D, E auch je nach Messergebnis grün bzw. rot gefärbt sein können, um das Messergebnis rasch zu visualisieren.

## Patentansprüche

1. Verfahren zum Überprüfen einer Passform einer mit einem lösbar befestigten, einen Schalldurchtritt regulierenden Element (2) ausgestatteten Otoplastik (1) in einem menschlichen Ohr bzw. Gehörgang, **dadurch gekennzeichnet, dass** das Verfahren folgende Schritte umfasst:
a) Entnehmen des Elementes (2) aus der Otoplastik (1);
b) Befestigen eines Messadapters (8) mit einem dem Ohr bzw. Gehörgang zugewandten Mikrofon (9) in einer Position des Elementes (2) derart, dass der Messadapter (8) lose sitzt und Schall zwischen dem Messadapter (8) und der Otoplastik (1) ungehindert durchtreten kann;
c) Beschallen der Otoplastik (1) und Aufnehmen eines Referenzspektrums mit zumindest einem Prüfsignal;
d) Befestigen des Messadapters (8) in der Otoplastik (1) derart, dass der Messadapter (8) fest anliegt und kein Schall zwischen dem Messadapter (8) und der Otoplastik (1) durchdringen kann;
e) Beschallen der Otoplastik (1) und Aufnehmen eines Messspektrums unter den gleichen Bedingungen wie beim Aufnehmen des Referenzspektrums;
f) Bilden einer Differenz aus dem Messspektrum abzüglich des Referenzspektrums und Ermitteln der Passform der Otoplastik (1) anhand der Differenz;
wobei das Messspektrum auch vor dem Referenzspektrum aufgenommen werden kann.

2. Verfahren nach Anspruch 1, **dadurch gekennzeichnet, dass** die Otoplastik (1) mit Schall einer Frequenz von weniger als 300 Hz beschallt wird.

3. Verfahren nach Anspruch 1 oder 2, **dadurch gekennzeichnet, dass** das Beschallen mittels eines Kopfhörers (7) durchgeführt wird.

4. Verfahren nach einem der Ansprüche 1 bis 3, **dadurch gekennzeichnet, dass** mehrere Prüfsignale verschiedener Frequenzen angewandt werden und aus der Differenz ein Mittelwert gebildet wird, der mit einem vorgegebenen Mittelwert bzw. Qualitätskriterium für die Passform der Otoplastik (1) verglichen wird.

5. Verfahren nach einem der Ansprüche 1 bis 4, **dadurch gekennzeichnet, dass** mehrere Prüfsignale verschiedener Frequenzen angewandt werden und für jede Frequenz eine Differenz ermittelt und mit einem vorgegebenen Sollwert bzw. Sollbereich verglichen wird.

## Claims

1. A method for checking the fit of an otoplastic (1), equipped with a releasably fastened element (2) regulating sound penetration, in a human ear or hearing passage, **characterised in that** the method comprises the following steps:
a) removing the element (2) from the otoplastic (1) ;
b) fastening a measurement adapter (8) to a microphone (9), facing the ear or hearing passage, in a position of the element (2), in such a way that the measurement adapter (8) sits loosely and sound can penetrate freely between the measurement adapter (8) and the otoplastic (1);
c) treating the otoplastic (1) with sound and recording a reference spectrum using at least one test signal;
d) fastening the measurement adapter (8) in the otoplastic (1) in such a way that the measurement adapter (8) fits rigidly against the otoplastic and no noise can penetrate between the measurement adapter (8) and the otoplastic (1);
e) treating the otoplastic (1) with sound and recording a measurement spectrum under the same conditions as when recording the reference spectrum;
f) forming a difference from the measurement spectrum less the reference spectrum and establishing the fit of the otoplastic (1) on the basis of the difference;
wherein the measurement spectrum can also be recorded before the reference spectrum.

2. The method according to claim 1, **characterised in that** the otoplastic (1) is treated with sound using sound of a frequency of less than 300 Hz.

3. The method according to claim 1 or 2, **characterised in that** the treatment with sound is carried out by means of headphones (7).

4. The method according to one of claims 1 to 3, **characterised in that** a plurality of test signals of different frequencies are applied and a mean value is formed from the difference, said mean value being compared to a predefined mean value or quality criterion for the fit of the otoplastic (1) .

5. The method according to one of claims 1 to 4, **characterised in that** a plurality of test signals of different frequency are applied and, for each frequency, a difference is established and is compared to a predefined target value or target range.

## Revendications

1. Procédé de contrôle d'une forme d'ajustage d'une otoplastique (1) équipée d'un élément (2) régulant un passage de son et fixé de manière amovible dans une oreille ou un couloir auditif humain, **caractérisé en ce que** ce procédé comprend les étapes suivantes:
a) retrait de l'élément (2) de l'otoplastique (1) ;
b) fixation d'un adaptateur de mesure (8) pourvu d'un micro (9) tourné vers l'oreille ou le couloir auditif dans une position de l'élément (2) telle que l'adaptateur de mesure (8) soit posé de manière lâche et que le son puisse passer sans obstacles entre l'adaptateur de mesure (8) et l'otoplastique (1) ;
c) sonorisation de l'otoplastique (1) et enregistrement d'un spectre de référence comportant au moins un signal de contrôle;
d) fixation de l'adaptateur de mesure (8) dans l'otoplastique (1) de manière à ce que l'adaptateur de mesure (8) soit posé de manière stable et à ce qu'aucun son ne puisse passer entre l'adaptateur de mesure (8) et l'otoplastique (1);
e) sonorisation de l'otoplastique (1) et enregistrement d'un spectre de référence dans les mêmes conditions que lors de l'enregistrement du spectre de référence;
f) calcul d'une différence entre le spectre de mesure et le spectre de référence et détermination de la forme d'ajustage de l'otoplastique (1) à partir de la différence;
le spectre de mesure pouvant aussi être enregistré avant le spectre de référence.

2. Procédé selon la revendication 1, **caractérisé en ce que** l'otoplastique (1) reçoit du son à une fréquence de moins de 300 Hz.

3. Procédé selon la revendication 1 ou 2, **caractérisé en ce que** la sonorisation est réalisée au moyen d'un écouteur porté sur la tête (7).

4. Procédé selon une des revendications 1 à 3, **caractérisé en ce que** plusieurs signaux de fréquences différentes sont appliqués et que, à partir de la différence, est calculée une valeur moyenne qui est comparée à une valeur moyenne ou un critère de qualité prédéfini pour la forme d'ajustage de l'otoplastique (1).

5. Procédé selon une des revendications 1 à 4, **caractérisé en ce que** plusieurs signaux de fréquences différentes sont appliqués et que, pour chaque fréquence, on détermine une différence et on la compare à une valeur théorique ou plage théorique prédéfinie.
